# EUROPÄISCHE PATENTSCHRIFT

(11) **EP 3 713 925 B1**
(45) Veröffentlichungstag und Bekanntmachung des Hinweises auf die Patenterteilung: **22.04.2026**
(21) Anmeldenummer: 18829725.3
(22) Anmeldetag: 21.11.2018
(51) Int. Cl.: C07D 311/30

(54) **SYNTHESE VON MORIN UND MORINDERIVATEN**
SYNTHESIS OF MORIN AND MORIN DERIVATIVES
SYNTHÈSE DE MORINE ET DE DÉRIVÉS DE LA MORINE

(30) Priorität: 21.11.2017 EP 17202835
(43) Veröffentlichungstag der Anmeldung: 30.09.2020
(73) Patentinhaber: Orsatec GmbH, 86399 Bobingen (DE)
(72) Erfinder: LINDEL, Thomas, 30163 Hannover (DE); MENDE, Steffen, 30126 Braunschweig (DE)
(74) Vertreter: Stolmár & Partner Patentanwälte PartG mbB
(86) Internationale Anmeldenummer: PCT/EP2018/000521
(87) Internationale Veröffentlichungsnummer: WO 2019/101353

(56) Entgegenhaltungen:
- JIANG WEN-JUN ET AL: "Stereospecific inhibition of nitric oxide production in macrophage cells by flavanonols: Synthesis and the structure-activity relationship", BIOORGANIC & MEDICINAL CHEMISTRY, ELSEVIER, AMSTERDAM, NL, vol. 23, no. 21, 28 September 2015 (2015-09-28), pages 6922 - 6929, XP029299000, ISSN: 0968-0896, DOI: 10.1016/J.BMC.2015.09.042
- B. VYAS; M. SINGH; M. KAUR; O. SILAKARI; M. S. BAHIA; B. SINGH, MED. CHEM. RES., vol. 25, 3 February 2016 (2016-02-03), pages 609 - 626, XP002781436
- SHENG XIAO ET AL: "The total synthesis of ( )-sanggenol F", TETRAHEDRON, vol. 73, no. 25, 7 May 2017 (2017-05-07), pages 3485 - 3491, XP085040659, ISSN: 0040-4020, DOI: 10.1016/J.TET.2017.05.022

## Beschreibung

Der Färbermaulbeerbaum *(Maclura tinctoria* oder auch *Morus tinctoria)* ist ein in Zentralamerika, der Karibik, Ostindien und im tropischen Südamerika heimischer Laubbaum, der eine durchschnittliche Höhe von 20-30 m und eine Stammdicke von 70-80 cm erreichen kann. Das Kernholz des Färbermaulbeerbaumes wird allgemein als Gelbholz bezeichnet und wurde schon in der Präkolumbischen Zeit zum Färben von Textilien verwendet. Die Farbstoffe im Gelbholz sind Maklurin (auch Maclurin oder Moringerbsäure) und das Pentahydroxyflavonol Morin der folgenden Formel I:

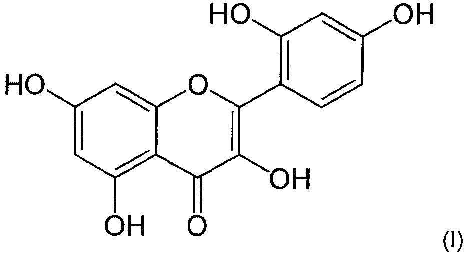

Morin (2,4-Dihydroxyphenyl)-3,5,7-trihydroxy-4H-chromen-4-on) gehört zur Gruppe der Flavonole, einer Unterklasse der Flavonoide. Der Name der meist gelben Pflanzenfarbstoffe kommt von lat. flavus = gelb. Morin besitzt fünf Hydroxygruppen und drei zyklische Systeme. Das Grundgerüst der Flavonole mit Nummerierung der Gerüstatome, zusammen mit dem strukturähnlichen Auronen und den offenkettigen Chalkonen, ist der Fig. 1 zu entnehmen.

Morin wirkt antioxidativ und entzündungshemmend und ist kaum toxisch. Somit erfüllt es eine wichtige Voraussetzung für pharmakologische Anwendungen. Die 3-Hydroxylierung bei Morin erhöht verglichen mit den in 3-Stellung unsubstituierten Flavonen dessen antioxidative Wirkung. Aufgrund seines niedrigen pKa-Werts von 3,5 (3-OH-Gruppe) liegt Morin unter physiologischen Bedingungen deprotoniert vor und bindet, anders als andere Flavonoide, kaum an negativ geladene DNA oder RNA. Morin (1) komplexiert als exzellenter anionischer Ligand Übergangsmetall- (z. B. 2:1-Komplex mit Zn²⁺)und Lanthanoid-Kationen (z. B. 3:1-Komplex mit La³⁺) unter Deprotonierung der 2'-OH- bzw. 3-OH-Gruppe und findet Anwendung in der fluorimetrischen Analytik diverser Metallionen. Mit Al³⁺ bildet sich ein grün fluoreszierender 3:1-Komplex.

Noch bis heute wird Morin für den kommerziellen Gebrauch aus dem Extrakt des Gelbholzes isoliert. Allerdings zeigt kommerziell erhältliches Morin (sowohl das natürlich wie auch das synthetisch erhaltene) eine Reinheit von nur etwa 85% mit zumeist der Nebenkomponente Kaempferol (II) als Verunreinigung, dem gegenüber Morin die 2'-Hydroxygruppe fehlt. Darüberhinaus gibt es weitere Verunreinigungen, die üblicherweise ein Gemisch aus Polyphenolen bzw. Flavonoiden sind. Diese Verunreinigungen, die nur extrem aufwändig zu entfernen sind oder gar nicht zu entfernen sind, stören bei vielen Anwendungen, sei es in der Pharmazie oder auch beim Elektroplating oder der Galvanik.

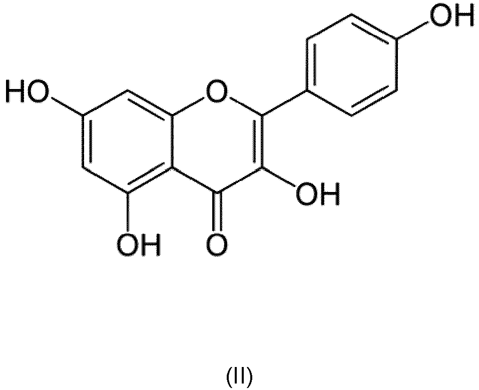

Somit besteht ein Bedarf, ein Verfahren zur direkten synthetischen Herstellung von hochreinem Morin und Morinderivaten bereitzustellen. Zudem handelt es sich bei Gelbholz um Tropenholz, dessen Verwendung aus Umweltschutzgründen nicht unbedenklich ist.

Im Labormaßstab werden im Stand der Technik zwei Synthesewege beschrieben. Obwohl die Struktur von Morin bereits 1896 durch Perkin aufgeklärt wurde und umfangreiche Literatur zur Synthese von Flavonoiden allgemein existiert, wurde Morin erst 2013 synthetisiert, wobei eine Allan-Robinson-Kondensation ausgehend von 2-Methoxy-1-(2,4,6-trihydroxyphenyl)ethan-1-on und 2,4-Dimethoxybenzoylchlorid genutzt wird (CN 103342690 A). Weiterhin wurde eine ähnliche Route zu Morin beschrieben, die über das Chalkon (E)-3-(2,4-Dimethoxyphenyl)-1-(2-hydroxy-4,6-dimethoxyphenyl)prop-2-en-1-on (8) verläuft (CN 105985306). Dabei wird die Aldolkondensation des Acetophenon-Derivats 1-(2-Hydroxy-4,6-dimethoxyphenyl)ethan-1-on mit 2,4-Dimethoxybenzaldehyd genutzt. B. Vyas, M. Singh, M. Kaur, O. Silakari, M. S. Bahia, B. Singh, Med. Chem. Res. 2016, 25, 609-626 beschreiben das Design und Screening von Aldosereduktaseinhibitoren, bei dem auch Morin als Reduktaseinhibitor untersucht wird. Ferner wird ein Weg zur Synthese von Morin beschrieben, der ohne die Methylierung der phenolischen Hydroxygruppen auskommen soll.

In Tetrahedron 73 (2017), 3485-3491 wird die Herstellung von Sanggenol-F ausgehend von 2,4,6-Trihydroxyacetophenon über 15 Stufen offenbart, bei der Morin als Zwischenprodukt gebildet wird. Weiterhin wird dort eine 7-Stufensynthese ausgehend von natürlich vorkommendem Morin beschrieben.

Aufgabe der vorliegenden Erfindung ist es, ein Verfahren zur Herstellung von hochreinem Morin und Morinderivaten bereitzustellen, welches auf leicht erhältlichen und preiswerten Ausgangsstoffen beruht, sowie die Bereitstellung von hochreinem synthetisch hergestelltem Morin und Morinderivaten, d. h. ohne die vorstehend erwähnten Verunreinigungen. Der Begriff Morinderivate wird vorliegend so verstanden, dass dieser direkt aus Morin herstellbare Abkömmlinge umfasst, sowie Verbindungen, die mindestens das Kohlenstoffgerüst von Morin samt Oxygenierungsmuster aufweisen, von großer Reinheit.

Die Aufgabe wird gelöst durch ein Verfahren zur Herstellung einer Verbindung mit der folgenden Formel (1), 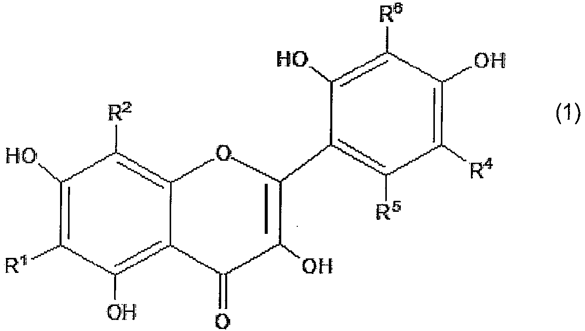 umfassend die Schritte:
i) Acetylierung einer Verbindung der folgenden Formel (4) 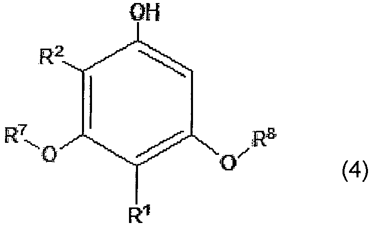 zur Bildung des Acetophenons der Formel (6), 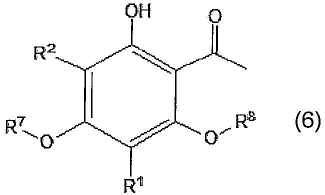
ii) Umsetzen des Acetophenons der Formel (6) mit einer Verbindung der folgenden Formel (7) unter basischen Bedingungen bei Raumtemperatur 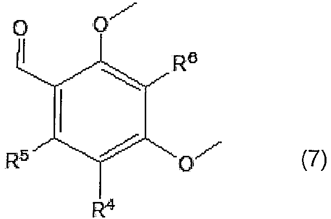 zur Bildung des Chalkons der folgenden Formel (8), 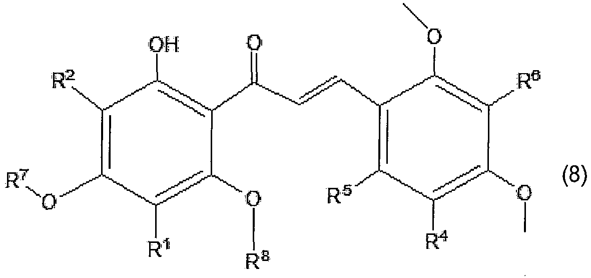
iii) Umsetzen des Chalkons der Formel (8) unter oxidierenden Bedingungen in basischem Milieu zur Bildung eines Flavonols mit der folgenden Formel (9); und 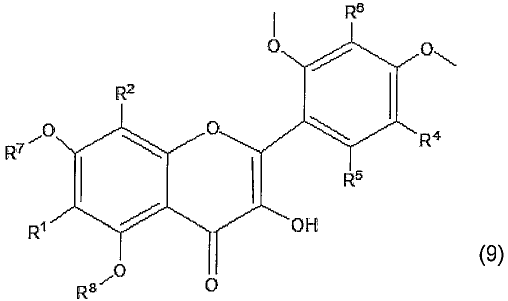
iv) Demethylierung des Flavonols der Formel (9),
wobei
R¹, R², R³, R⁴, R⁵ und R⁶ unabhängig voneinander ein verzweigtes oder unverzweigtes C₁-C₈₋Alkyl, NO₂, SO₃H, NX₂, wobei X ein Ethyl- oder Methylrest oder Wasserstoff ist, CF₃ oder Wasserstoff sind, bevorzugt C₁-C₅-Alkyl oder Wasserstoff, am meisten bevorzugt C₁-C₃-Alkyl oder Wasserstoff, und
R⁷ und R⁸ unabhängig voneinander eine Methyl-, Ethyl-, tert.-Butyl-, Benzyl-, Methoxymethyl-, *p*-Methoxybenzyl-, Benzyloxymethyl-, Triphenylmethyl-, Tetrahydropyranyl- oder Allyl-Gruppe sind.

Das Verfahren beruht dabei auf leicht erhältlichen Ausgangsstoffen und bietet eine hohe Ausbeute für das gewünschte Produkt. Zudem wird das Produkt in hoher Reinheit gewonnen, welches somit eine höhere Aktivität als natürlich gewonnenes Morin zeigt.

Das Verfahren kann zusätzlich oder alternativ dazu einen Schritt
(i-a) Methylierung einer Verbindung der folgenden Formel (3) 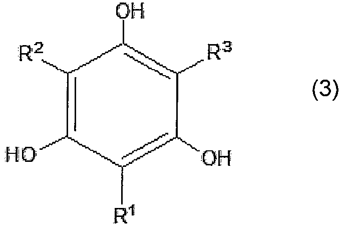 unter sauren Bedingungen zur Bildung einer Verbindung der Formel (4) umfassen, wobei R¹, R², R³, R⁴, R⁵ und R⁶ wie in oben definiert sind.

Hierdurch können Morin bzw. Morinderivate aus weiteren, leicht erhältlichen Ausgangsstoffen synthetisiert werden, z.B. dem kommerziell erhältlichen Trimethoxybenzol, das direkt eingesetzt werden kann.

In einer bevorzugten Ausführungsform ist R³ Wasserstoff. In einer besonders bevorzugten Ausführungsform sind R¹, R², R³, R⁴, R⁵ und R⁶ Wasserstoff. Somit wird durch das erfindungsgemäße Verfahren besonders bevorzugt hochreines Morin der Formel I erhalten:

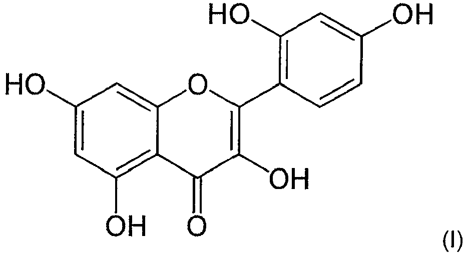

In Schritt iii) können die oxidierenden Bedingungen bevorzugt mittels tert.-Butylhydroperoxid und das basische Milieu bevorzugt mit Hilfe eines Alkalihydroxids hergestellt werden. Alkalihydroxide sind dabei LiOH, KOH und NaOH. Die Verwendung von *tert.-*Butylhydroperoxid vermindert die Bildung von unerwünschten Nebenprodukten mit Auron-Gerüst und erhöht zudem die Ausbeute.

In Schritt ii) können die basischen Bedingungen mit Hilfe eines Alkalihydroxids hergestellt werden. Zusätzlich kann die Reaktion bei Raumtemperatur stattfinden.

In Schritt i) kann die Acetylierung mit Hilfe von Acetylchlorid in Anwesenheit einer Lewis-Säure und Dichlormethan erfolgt. Lewis-Säuren können hierbei bevorzugt Bortrichlorid, BBr₃ oder AlCl₃ oder Mischungen der vorgenannten sein, ganz besonders ist AlCl₃ oder Bortrichlorid bevorzugt.

In Schritt (i-a) können die sauren Bedingungen mit Hilfe einer anorganischen Säure hergestellt werden. Anorganische Säure sind hierbei bevorzugt Salpetersäure, Salzsäure oder Schwefelsäure.

Weiterhin wird die Aufgabe gelöst durch die Bereitstellung einer kaempferolfreien Verbindung mit der Formel (1), erhältlich durch das erfindungsgemäße Verfahren. In einer besonders bevorzugten Ausführungsform sind in der Verbindung der Formel (1) R¹, R², R³, R⁴, R⁵ und R⁶ Wasserstoff, d.h die Verbindung ist kaempferolfreies Morin der Formel (I). Das erfindungsgemäße Morin zeichnet sich durch eine sehr hohe Reinheit aus; bevorzugt beträgt die Reinheit mehr als 85%, 90% oder ganz besonders bevorzugt mehr als 95%, in besonders bevorzugten Ausführungsformen wird eine Reinheit von 99% erzielt. Reinheit bedeutet vorliegend, dass Polyphenole bzw. Flavonoide nur in den vorgenannten Mengen nachweisbar sind. Insbesondere ist wie schon gesagt das Morin gemäß der vorliegenden Erfindung frei von Kaempferol Verunreinigungen d.h. frei von Kaempferol der Formel (II) und/oder seinen Derivaten. Vorliegend bedeutet der Ausdruck "frei von", dass im Rahmen der üblichen Meßgenauigkeit Kaempferol bzw. dessen Derivate in einem Anteil von weniger 0,5 %, in spezifischen Ausgestaltungen von weniger als 0,3 % vorhanden sind. In besonders bevorzugten Ausführungsformen ist Kaempferol bzw. dessen Derivate im Rahmen der Meßgenauigkeit nicht nachweisbar. Das hochreine Morin erhältlich gemäß der vorliegenden Erfindung kann z.B. direkt ohne weitere Aufreinigung bei der elektrolytischen Zinn- oder Zinn/Bleiabscheidung (z.B. gemäß der EP 810303 A1 oder der DE 19623274 A1) eingesetzt werden. Es wurde gefunden, dass die Aktivität des Morins in der Sn und Sn/Pb Abscheidung nicht linear mit dem Gehalt an Morin in dem bislang verwendeten Naturprodukt korreliert, was auf die nicht entfernbaren Verunreinigungen, insbesondere Kaempferol und Polyphenole und Flavonoide, zurückzuführen ist. Man nimmt an, dass natürliche, technisch bislang nicht entfernbare Verunreinigungen die Aktivität des Morins absenken. Das reine Syntheseprodukt gemäß der vorliegenden Erfindung enthält diese natürlichen Verunreinigungen nicht und zeigt daher eine gegenüber dem Naturprodukt um 22,35% verbesserte Aktivität. Verbesserte Aktivität im Sinne der vorliegenden Erfindung bedeutet dabei eine um diesen Prozentsatz vergrößerte Bedeckung eines Bleches mit Sn oder Sn/Pb in einer galvanotechnischen sogenannten Hull-Zelle wie es in der EP 810303 A1 detailliert beschrieben wird. Darüberhinaus ist die Porosität der mittels des erfindungsgemäßen Morins erhaltenen Zinn bzw. Zinn/Bleischicht (gemessen mittels Feuchtetransport FSP, Diss. H. Künzel, Univ. Stuttgart 1994) um ca. 35% geringer als bei dem Vergleichsbeispiel, das mittels des natürlichen Morins hergestellt wurde. Dabei wurde festgestellt, dass unabhängig von der vorliegend getesteten Bezugsquelle (Sigma-Aldrich, Adooq Bioscience, Aurantika, Fisher-Scientific) des natürlichen Morins, die verbesserten Eigenschaften immer in der gleichen Größenordnung (im Rahmen der üblichen Messtoleranzen) sind.

Die Erfindung ist nachstehend anhand von Beispielen erläutert,

### Beispiel 1

### Herstellung von 3,5-Dimethoxyphenol (4)

Unter Argonatmosphäre wurde Phloroglucinol (3) (80.0 g, 634 mmol, 1.0 Äq.) in Methanol (400 mL) gelöst. Es wurde langsam Schwefelsäure (95%, 46.3 mL, 825 mmol, 1.3 Äq.) zugetropft, und das Reaktionsgemisch wurde für 26 h bei 80 °C bei Rückfluss gerührt. Im Anschluss wurden 10proz. wässrige Kaliumcarbonat-Lösung (800 mL) und Toluol (300 mL) zugegeben. Nach Separieren der Phasen wurde die wässrige Phase zweimal mit Toluol (je 300 mL) extrahiert. Die wässrige Phase wurde mit 2 M HCl-Lösung auf pH<2 gebracht und dreimal mit Ethylacetat (je 300 mL) extrahiert. Die vereinigten organischen Phasen wurden über Natriumsulfat getrocknet und das Lösungsmittel im Hochvakuum entfernt. Nach Aufreinigung mittels fraktionierter Destillation wurde Verbindung (4) (81.2 g, 527 mmol, 83%) als farbloses Öl erhalten. Zusätzlich konnte das Nebenprodukt (5) (4.71 g, 28.0 mmol, 4.4%) als farbloses Öl erhalten werden. 3,5-Dimethoxyphenol (4): Sdp.: 139-141 °C (2.8 mbar). DC [Petrolether/Ethylacetat (2:1)]: *R*_{f} = 0.40. ¹H-NMR (400 MHz, CDCl₃): δ = 6.08 (t, *J* = 2.2 Hz, 1H, 4-CH), 6.03 (d, *J* = 2.2 Hz, 2H, 2-CH, 6-CH), 5.19 (s, 1H, OH), 3.75 (s, 6H, OCH₃). ¹³C-NMR (100 MHz, CDCl₃): δ = 162.0 (2C, C_{Ph}-OCH₃), 157.7 (C_{Ph}-OH), 94.6 (2C, C-2, C-6), 93.5 (C-4), 55.7 (2C, OCH₃). IR (Diamant-ATR): *ṽ* = 3384 (m, br.),3003 (w), 2942 (w), 2842 (w), 1595 (s), 1500 (m), 1458 (m), 1433 (m), 1342 (m), 1294 (m), 1192 (s), 1139 (s), 1052 (s), 992 (m), 973 (m), 924 (m), 818 (s), 679 (m). UV (MeOH): λₘₐₓ (lg ε) = 267 (2.79), 206 (4.60). MS (EI): *m*/*z* (%) = 69 [M - MeOC-CH-COH]⁺ (18), 125 [M - COH]⁺ (60), 154 [M]⁺ (100).

Das Produkt konnte in einer Ausbeute von 78% erhalten werden. Dies wurde vor allem aufgrund der zweiten Extraktion der wässrigen Phase nach dem Verringern des pH-Wertes möglich, wodurch zusätzlich 23% Ausbeute des Produkts dazugewonnen werden. Eine Erhöhung der Reaktionszeit war vor allem bei größeren Ansätzen notwendig; so wurde diese von 21 h bei 500 mg- auf 30 h bei 20 g-Ansätzen erhöht. Die Charakterisierungsanalysen des Produkts deckten sich mit den Erwartungen.

Bei der Reaktion konnten zwei Nebenprodukte gefunden und isoliert werden. Bei einem davon handelt es sich um das einfach methylierte 5-Methoxybenzol-1,3-diol, was durch ¹H-NMR und ¹³C-NMR-Analysen bestätigt werden konnte. Die Ausbeute lag hier bei 15%. Das andere Nebenprodukt ist das dreifach methylierte 3,5-Trimethoxybenzol, welches in einer Ausbeute von 7% auftrat. Auch hier konnten ¹H-NMR und ¹³C-NMR-Analysen die Struktur bestätigen. Nebenprodukt 1,3,5-Trimethoxybenzol (5) [12]: Ausbeute: 7%. DC [Petrolether/Ethylacetat (2:1)]: *R*_{f} = 0.78. Sdp.: 135-137 °C (2.8 mbar). ¹H-NMR (400 MHz, CDCl₃): δ = 6.09 (s, 3H, Ph-H), 3.77 (s, 9H, OCH₃). ¹³C-NMR (100 MHz, CDCl₃): δ = 161.6 (3C, C_{Ph}-OCH₃), 92.9 (3C, C_{Ph}), 55.3 (3C, OCH₃). IR (Diamant-ATR): *ṽ* = 3075 (w), 3004 (m), 2963 (m), 2940 (m), 2839 (m), 1591 (s), 1480 (m), 1456 (s) 1423 (m), 1339(m), 1322 (m), 1252 (w), 1209 (s), 1194 (m), 1147 (s), 1064 (s), 1034 (s), 990 (m), 942 (m), 916 (m), 847 (s), 822 (s), 779 (s), 686 (s), 640 (m), 616 (m), 591 (m), 537 (m). UV (MeOH): λₘₐₓ (Ig ε) = 266 (2.62), 207 (4.47). MS (EI): *m*/*z* (%) = 69 [M - MeOC-CH-COMe]⁺ (83), 125 [M - COMe]⁺ (100), 167 [M]⁺ (100).

### Beispiel 2

### Herstellung von 1-(2-Hydroxy-4,6-dimethoxyphenyl)ethan-1-on (6)

Unter Argonatmosphäre wurde Bortrichlorid (1 M in Dichlormethan, 48.9 mL,48.9 mmol, 1.0 Äq.) in Dichlormethan (50 mL) gelöst und auf -10 °C gekühlt. Es wurde 3,5-Dimethoxyphenol (4) (7.54 g, 48.9 mmol, 1.0 Äq.), in Dichlormethan (25 mL) vorgelöst, zugetropft. Das Reaktionsgemisch wurde auf Raumtemperatur gebracht und für 30 min gerührt. Anschließend wurde innerhalb von 15 min Acetylchlorid (4.54 mL, 63.6 mmol, 1.3 Äq.), vorgelöst in Dichlormethan (80 mL), zugetropft. Das Reaktionsgemisch wurde für 3 h unter Rückfluss gerührt. Nach dem Abkühlen wurde 1 M wässrige HCl-Lösung (300 mL) zugegeben. Die Phasen wurden getrennt und die wässrige Phase dreimal mit Ethylacetat (je 200 mL) extrahiert. Die vereinigten organischen Phasen wurden über Natriumsulfat getrocknet und das Lösungsmittel im Hochvakuum entfernt. Nach säulenchromatographischer Reinigung an Kieselgel [Petrolether/Ethylacetat (2:1)] wurde das Acetophenon-Derivat (6) (7.38 g, 37.6 mmol, 77%) als farbloser Feststoff erhalten. DC [Petrolether/Ethylacetat (2:1)]: *R*_{f} = 0.73. Schmp.: 79-82 °C. ¹H-NMR (400 MHz, DMSO-*d*₆): δ = 14.03 (s, 1H, OH), 6.06 (d, *J* = 2.4 Hz, 1H, 5-CH), 5.92 (d, *J* = 2.4 Hz, 1H, 3-CH), 3.85 (s, 3H, *o*-Ph-OCH₃), 3.82 (s, 3H, *p*-Ph-OCH₃), 2.61 (s, 3H, O=CCH₃). ¹³C-NMR (100 MHz, DMSO-*d*₆): δ = 203.2 (C=O), 167.6 (*p*-C_{Ph}-OCH₃), 166.1 (o-C_{Ph}-OCH₃), 162.9 (o-C_{Ph}-OH), 106.0 (C_{Ph}-C=O), 93.5 (C-5), 90.75 (C-3), 55.5 (2C, OCH₃),32.9 (CH₃). IR (Diamant-ATR): *ṽ* = 3103 (w, br.), 3008 (w), 2945 (w, br.), 2849 (w, br.), 2705 (w, br.),2599 (w, br.), 1613 (s), 1457 (m), 1441 (m), 1423 (m), 1389 (m), 1366 (m), 1325 (m), 1269 (s), 1220 (s),1205 (s), 1155 (s), 1112 (m), 1081 (m), 1045 (m), 1030 (m), 962 (m), 941 (m), 893 (m), 835 (s), 806 (m),744 (m), 715 (w), 690 (w), 657 (m), 628 (w), 596 (m), 557 (m), 531 (m). UV (MeOH): λₘₐₓ (lg ε) = 286(4.26), 209 (4.21). MS (ESI): *m*/*z* (%) = 197 [M + H]⁺ (49), 219 [M + Na]⁺ (100). HRMS (ESI+): *m*/*z* = 219.06279 (0.1 ppm, ber.: 219.06278 [M+Na]⁺).

### Beispiel 3

### Herstellung von 3-(2,4-Dimethoxyphenyl)-1-(2-hydroxy-4,6-dimethoxyphenyl)prop-2-en-1-on (8)

Unter Argonatmosphäre wurden das Acetophenon-Derivat (6) (4.80 g, 24.5 mmol, 1.0 Äq.) und 2,4-Dimethoxybenzaldehyd (7) (6.01 g, 36.7 mmol, 1.5 Äq.) in Ethanol (150 mL) gelöst. Eine Lösung von NaOH (8.80 g, 220 mmol, 9.0 Äq.) in dest. Wasser (38 mL) wurde zugetropft. Das Reaktionsgemisch wurde für 117 h bei Raumtemp. gerührt. Im Anschluss wurde 1 M wässrige HCl-Lösung (350 mL) hinzugegeben und für weitere 15 min gerührt. Die Lösung wurde dreimal mit Ethylacetat (je 250 mL) extrahiert. Die vereinigten organischen Phasen wurden über Natriumsulfat getrocknet und das Lösungsmittel im Hochvakuum entfernt. Nach säulenchromatographischer Reinigung an Kieselgel [Petrolether/Ethylacetat (3:1 →1:1)] wurde das Chalkon (8) (5.87 g, 17.0 mmol, 70%) als gelber Feststoff erhalten. DC [Petrolether/Ethylacetat (4:1)]: *R*_{f} = 0.32. Schmp.: 126-129 °C. ¹H-NMR (400 MHz, CDCl₃) δ = 14.54 (s, 1H, 2'-OH), 8.10 (d, *J* = 15.7 Hz, 1H, β-H), 7.90 (d, *J =* 15.7 Hz, 1H, α-H), 7.54 (d, *J =* 8.6 Hz, 1H, 6-H), 6.53 (dd, *J* = 8.6, 2.4 Hz, 1H, 5-H), 6.47 (d, *J* = 2.4 Hz, 1H, 3-H), 6.10 (d, *J = 2.4* Hz, 1H, 3'-H), 5.95 (d, *J* = 2.4 Hz, 1H, 5'-H), 3.90 (s, 3H, 6'-OCH₃), 3.89 (s, 3H, 2-OCH₃), 3.85 (s, 3H, 4- OCH₃),3.83 (s, 3H, 4'-OCH₃). ¹³C-NMR (100 MHz, CDCl₃): δ = 193.0 (C=O), 168.3 (C -6'), 165.8 (C-4'), 162.8 (C-4), 162.4 (C-2'), 160.2 (C-2), 138.3 (C-β), 130.4 (C-6), 125.3 (C-α), 117.8 (C-1), 106.5 (C-1'), 105.5 (C-5), 98.4 (C -3), 93.8 (C-5'), 91.1 (C-3'), 55.7 (2'-OCH₃), 55.5 (2- OCH₃), 55.5 (4'-OCH₃), 55.4 (4- OCH₃). IR (Diamant-ATR): *ṽ* = 3120 (w, br.), 3082 (w, br.), 3000 (w), 2942 (m, br.), 2840 (w, br.), 1604 (s), 1547 (s), 1502 (s), 1451 (m), 1437 (m), 1414 (m), 1343 (m), 1315 (m), 1293 (m), 1268 (s), 1206 (s), 1158 (s), 1106 (s), 1057(m), 1028 (s), 980 (m), 940 (m), 867 (m), 849 (m), 814 (s), 795 (s), 767 (m), 720 (m), 697 (m), 674 (m),648 (m), 621 (m), 604 (m), 582 (m), 563 (m). UV (MeOH): λₘₐₓ (Ig ε) = 379 (4.52), 251 (3.95), 207 (4.60).MS (EI): *m*/*z* (%) = 345 [M + H]⁺ (47), 367 [M + Na]⁺ (67), 711 [2M + Na]⁺ (100). HRMS (ESI+): *m*/*z* = 711.24176 (0.1 ppm, ber.: 711.24120 [2M+Na]⁺).

### Beispiel 4

### Herstellung von 2-(2,4-Dimethoxyphenyl)-3-hydroxy-5,7-dimethoxy-4H-chromen-4-on (9)

Unter Argonatmosphäre wurde das Chalkon (8) (1.00 g, 2.90 mmol, 1.0 Äq.) in Ethanol (40 mL) gelöst. Es wurde eine Lösung von *tert*.-Butylhydroperoxid (70% in H₂O, 4.0 mL, 29.0 mmol, 10 Äq.) und NaOH (1.74 g, 43.6 mmol, 15 Äq.) in dest. H₂O (4 mL) zugegeben und für 23 h bei Raumtemp. gerührt. Im Anschluss wurde wässrige HCl-Lösung (1.0 M, 100 mL) hinzugegeben und dreimal mit Ethylacetat (je 80 mL) extrahiert. Die vereinigten organischen Phasenwurden über Natriumsulfat getrocknet und das Lösungsmittel im Hochvakuum entfernt. Nach säulenchromatographischer Reinigung an Kieselgel [Petrolether/Ethylacetat (1:4)] wurde das Flavonol (9) (395 mg, 1.10 mmol, 38%) als gelblicher Feststoff erhalten, zusätzlich zum Auron (10) (44 mg, 0.13 µmol, 4.5%). Ebenso wurde das hydroxylierte Auron-Derivat (11) erhalten (118 mg, 329 µmol, 11%). DC [Petrolether/Ethylacetat (1:4)]: *R*_{f} = 0.45. Schmp.: 110-112 °C. ¹H-NMR (600 MHz, CDCl₃): δ = 7.50(d, *J* = 8.4 Hz, 1H, 6'-CH), 6.78 (s, 1H, OH), 6.61 (dd, *J* = 8.4, 2.4 Hz, 1H, 5'-CH), 6.58 (d, *J* = 2.3 Hz, 1H, 3'-CH), 6.48 (d, *J* = 2.3 Hz, 1H, 8-CH), 6.34 (d, *J* = 2.3 Hz, 1H, 6-CH), 3.97 (s, 3H, 4'-OCH₃), 3.87 (s, 6H, 2'-OCH3, 7-OCH₃), 3.84 (s, 3H, 5-OCH₃). ¹³C-NMR (150 MHz, CDCl₃): δ = 172.0 (C-4), 164.1 (C-7), 162.7 (C-4'), 160.6 (C-5), 159.6 (8C-C-1O), 158.8 (C-2'), 142.7 (C-2), 138.6 (C-3), 131.9 (C-6'), 112.4 (C-1'), 106.8(4C-C-5C), 104.8 (C-5'), 99.2 (C-3'), 95.6 (C-6), 92.6 (C-8), 56.4 (4'- OCH₃), 55.9 (5-OCH₃), 55.8 (2'- OCH₃),55.5 (7- OCH₃). IR (Diamant-ATR): *ṽ* = 3204 (m, br.), 3006 (w), 2961 (m), 2938 (m), 2840 (w), 1728 (m),1658 (m), 1601 (s), 1499 (m), 1461 (m), 1435 (m), 1414 (m), 1373 (m), 1317 (m), 1299 (m), 1281 (m),1252 (m), 1206 (s), 1161 (s), 1120 (m), 1092 (m), 1024 (m), 1002 (m), 967 (m), 936 (m), 918 (m), 873(m), 814 (m), 742 (w), 677 (w), 639 (m), 599 (m), 552 (m). UV (MeOH): λₘₐₓ (lg ε) = 338 (4.07), 286(3.89), 246 (4.32), 203 (4.64). MS (ESI): *m*/*z* (%) = 359 [M + H]⁺ (40), 381 [M + Na]⁺ (56), 739 [2M + Na]⁺(100). HRMS (ESI+): *m*/*z* = 739.20013 (0.02 ppm, ber.: 739.20028 [2M+Na]⁺).

Nebenprodukt 2-(2,4-Dimethoxybenzyliden)-4,6-dimethoxybenzofuran-3(2H)-on (10): DC [Petrolether/Ethylacetat (1:4)]: *R*_{f} = 0.44. ¹H-NMR (400 MHz, CDCl₃): δ = 8.19 (d, *J* = 8.7 Hz, 1H, 6'-H), 7.27 (s, 1H, 2C=CH), 6.58 (dd, *J* = 8.7, 2.4 Hz, 1H, 5'-H), 6.45 (d, *J* = 2.4 Hz, 1H, 3'-H), 6.36 (d, *J* = 1.9 Hz, 1H, 7H), 6.11 (d, *J* = 1.9 Hz, 1H, 5H), 3.94 (s, 3H, 4-OCH₃), 3.90 (s, 3H, 6-OCH₃), 3.87 (s, 3H, 2'-OCH₃),3.86 (s, 3H, 4'-OCH₃). ¹³C-NMR (100 MHz, CDCl₃): δ = 180.6 (C-3),168.6 (7-C-C-10), 168.4 (C-6), 162.2 (C-4'), 160.1 (C-2'), 159.3 (C-4), 146.8 (C-2), 132.8 (C-6'), 114.7 (C-1'), 105.7 (3C-C-4C), 105.5 (C-5'), 105.3 (2C=C), 98.0 (C-3'), 93.8 (C-5), 89.1 (C-7), 56.2 (4-OCH₃), 56.0 (6-OCH₃), 55.6 (2'-OCH₃), 55.4 (4'-OCH₃). IR (Diamant-ATR): *ṽ* = 2975 (w, br.), 2945 (w, br.), 2836 (w, br.), 1689 (m), 1648 (m), 1590 (s, br.), 1503 (m), 1447 (m), 1419 (m), 1362 (m), 1350 (m), 1321 (m),1289 (m), 1243 (s), 1217 (s), 1201 (s), 1155 (s), 1087 (s), 1033 (s), 946 (m), 917 (m), 890 (m), 815 (s),788 (s), 720 (m), 695 (m), 674 (m), 633 (m), 589 (m), 548 (m). UV (MeOH): λₘₐₓ (lg ε) = 403 (4.43), 252(3.93), 203 (4.53). MS (ESI): *m*/*z* (%) = 343 [M + H]⁺ (61), 365 [M+ Na]⁺ (100), 707 [2M + Na]⁺ (97). HRMS (ESI+): *m*/*z* = 365.09975 (0.5 ppm, ber.: 365.09956 [M+Na]⁺).

Nebenprodukt (*E*)-2-((2,4-Dimethoxyphenyl)(hydroxy)methylen)-4,6-dimethoxybenzofuran-3(2*H*)-on (11): DC [Petrolether/Ethylacetat (1:4)]: *R*_{f} = 0.63. Schmp.: 177-180 °C. ¹H-NMR (400 MHz, CDCl₃): δ = 9.47 (s, OH), 7.19 (d, *J* = 7.3 Hz, 6'-H), 6.57 (dd, *J* = 7.3, 2.1 Hz, 5'-H), 6.56 (d, *J* = 2.1 Hz, 3'-H), 6.53 (d, *J =* 2.3 Hz, 7-*H*), 6.36 (d, *J* = 2.3 Hz, 4-H), 3.99 (s, 3H, 3-COCH₃), 3.86 (s, 3H, 5- COCH₃),3.83 (s, 3H, 4'- COCH₃), 3.79 (s, 3H, 2'- COCH₃). ¹³C-NMR (100 MHz, CDCl₃): δ = 170.72 (3-C), 162.87 (6-C), 161.80 (2-C), 160.85 (4'-C), 158.67 (2'-C), 157.01 (4-C), 155.80 (7-C-C-1-0), 132.46 (6'-C), 112.98 (C-OH), 104.70 (5'-C), 100.41 (1'-C), 99.15 (3'-C), 99.09 (3-C-C-4-C),95.37 (5-C), 94.26 (7-C), 56.86 (4-C-CH₃), 55.86 (6-C-CH₃), 55.77 (2'-C-CH₃), 55.68 (4'-C-CH₃). IR (Diamant-ATR): *ṽ* = 3319 (m, br.), 3211 (w, br.), 3004 (m), 2951 (m, br.), 2841 (m), 2678 (w, br.), 1703(m), 1603 (s), 1576 (s), 1510 (m), 1446 (m), 1368 (m), 1281 (m), 1256 (m), 1204 (s), 1155 (s), 1110 (s), 1027 (s), 935 (m), 817 (m), 750 (m), 686 (m), 636 (m), 617 (m). UV (MeOH): λₘₐₓ (lg ε) = 621 (1.71), 314(3.71), 295 (3.72), 253 (3.86), 206 (4.39), 203 (4.39). MS (ESI): *m*/*z* (%) = 381 [M + Na]⁺ (100), 739 [2M+ Na]⁺ (42). HRMS (ESI+): *m*/*z* = 381.09460 (1.10 ppm, ber.: 381.09502 [M+Na]⁺).

### Beispiel 5

### Herstellung von 2-(2,4-Dihydroxyphenyl)-3,5,7-trihydroxy-4H-chromen-4-on (1, Morin)

Unter Argonatmosphäre wurde das Flavonol (9) (400 mg, 1.12 mmol, 1.0 Äq.) in Essigsäure (99%, 10 mL) gelöst und HBr (48% in H₂O, 50 mL) zugegeben. Anschließend wurde für 24 h zum Rückfluss erhitzt. Das Rohprodukt wurde vom Lösungsmittel weitestgehend befreit und in Ethanol (2 mL) aufgenommen. Nach Zugabe von Petrolether(100 mL), Filtration und Trocknung des Rückstands am Hochvakuum wurde Morin ((1), 220 mg, 728 µmol, 65%) als dunkelroter Feststoff erhalten. RP-DC [Wasser/Methanol (1:1)]: *R*_{f} = 0.35. Schmp.:> 250 °C. ¹H-NMR (400 MHz, DMSO-*d*₆): δ = 12.64 (s, 5-C-OH, 1H), 10.72 (s, COH, 1H), 9.79 (s, 4'-COH, 1H), 9.35 (s, COH, 1H), 8.89 (s, COH, 1H), 7.24 (d, *J* = 8.4 Hz, 6'-CH, 1H), 6.41 (d, *J =* 2.1 Hz, 3'-CH, 1H),6.36 (dd, *J* = 8.5, 2.3 Hz, 5'-CH, 1H), 6.30 (d, *J* = 2.1 Hz, 8-CH, 1H), 6.18 (d, *J* = 2.0 Hz, 6-CH, 1H). ¹³C-NMR (100 MHz, DMSO-*d*₆): δ = 176.2 (C-4), 163.7 (C-7), 160.9 (C-5), 160.4 (C-4'), 156.8 (2C, C-2', C-8a), 149.0 (C-2), 136.2 (C-3), 131.7 (C-6'), 109.3 (C-1'), 106.8 (C-5'), 103.6 (C-4a), 103.0 (C-2'), 98.0 (C-6), 93.4 (C-8). IR (Diamant-ATR): *ṽ* = 3212 (m, br.), 2731 (m, br.), 2341 (m, br.), 2116 (w, br.), 1995 (w, br.), 1920(w, br.), 1655 (m), 1626 (m), 1594 (m), 1570 (m), 1515 (m), 1480 (m), 1412 (m), 1365 (m), 1311 (m),1263 (m), 1224 (m), 1167 (s), 1102 (m), 1080 (m), 1011 (m), 983 (m), 969 (m), 874 (m), 833 (m), 805(m), 793 (m), 730 (m), 703 (m), 689 (m), 651 (m), 635 (m), 618 (m), 579 (m), 566 (m), 543 (m). UV (MeOH): λₘₐₓ (lg ε) = 372 (4.12), 263 (4.25), 205 (4.59). MS (ESI): *m*/*z* (%) = 301 [M - H]- (100), 303 [M+ H]+ (24), 325 [M + Na]⁺ (100), 627 [2M + Na]⁺ (23), 739 [2M + Na]⁺ (100). HRMS (ESI+): *m*/*z* = 325.03212 (0.92 ppm, ber.: 325.03242 [M+Na]⁺).

## Patentansprüche

1. Verfahren zur Herstellung einer Verbindung mit der folgenden Formel (1), 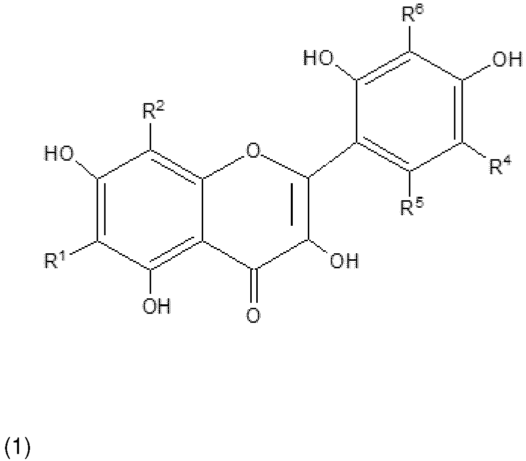 umfassend die Schritte:
i) Acetylierung einer Verbindung der folgenden Formel (4) zur Bildung des 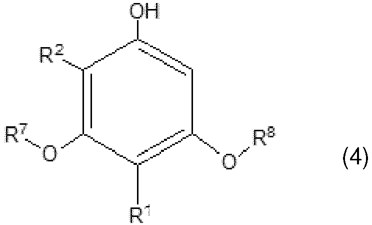 Acetophenons der Formel (6), 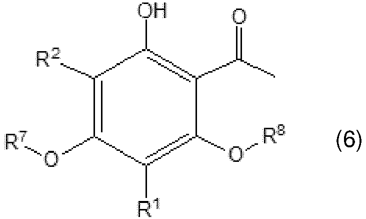
ii) Umsetzen des Acetophenons der Formel (6) mit einer Verbindung der folgenden Formel (7) unter basischen Bedingungen 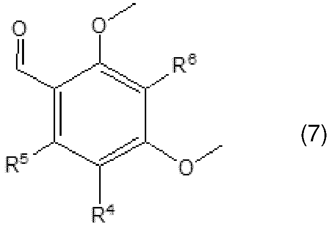 bei Raumtemperatur zur Bildung des Chalkons der folgenden Formel (8), 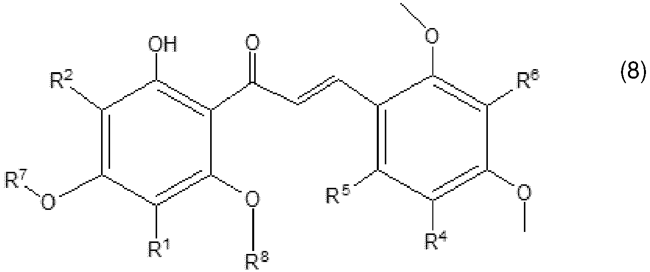
iii) Umsetzen des Chalkons mit der Formel (8) unter oxidierenden Bedingungen in basischem Milieu zur Bildung eines Flavonols mit der folgenden Formel (9); 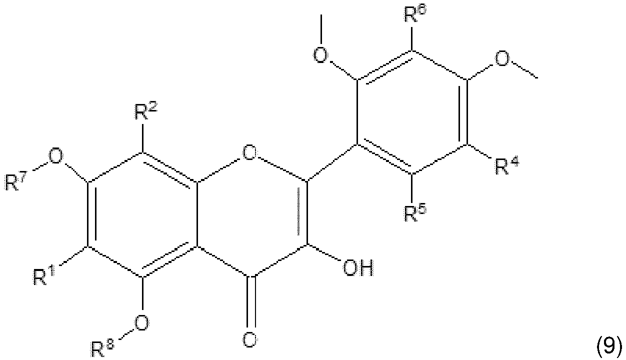
iv) und Demethylierung des Flavonols mit der Formel (9),
wobei
R¹, R², R³, R⁴, R⁵ und R⁶ ein verzweigtes oder unverzweigtes C₁-C₈₋Alkyl, NO₂, SO₃H, NX₂, wobei X ein Ethyl- oder Methylrest oder Wasserstoff ist, CF₃ oder Wasserstoff sind, bevorzugt C₁-C₅-Alkyl oder Wasserstoff, am meisten bevorzugt C₁-C₃-Alkyl oder Wasserstoff, und
R⁷ und R⁸ unabhängig voneinander eine Methyl-, Ethyl-, t-Butyl-, Benzyl-, Methoxymethyl-, p-Methoxybenzyl-, Benzyloxymethyl-, Triphenylmethyl-, Tetrahydropyranyl- oder Allyl-Gruppe sind.

2. Verfahren gemäß Anspruch 1, weiterhin umfassend den Schritt (i-a):
(i-a) Methylierung einer Verbindung der folgenden Formel (3) 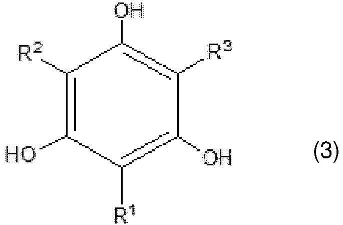
unter sauren Bedingungen zu einer Verbindung der Formel (4),
wobei R¹, R², R³, R⁴, R⁵ und R⁶ wie in Anspruch 1 definiert sind.

3. Verfahren gemäß einem der Ansprüche 1 bis 2, wobei R³ Wasserstoff ist.

4. Verfahren gemäß einem der Ansprüche 1 bis 3, wobei R¹, R², R³, R⁴, R⁵ und R⁶ Wasserstoff sind.

5. Verfahren gemäß einem der Ansprüche 1 bis 4, wobei in Schritt iii) die oxidierenden Bedingungen mit Hilfe von tert.-Butylhydroperoxid und das basische Milieu mit Hilfe eines Alkalihydroxids hergestellt werden.

6. Verfahren gemäß einem der Ansprüche 1 bis 5, wobei in Schritt ii) die basischen Bedingungen mit Hilfe eines Alkalihydroxids hergestellt werden und die Reaktion bei Raumtemperatur stattfindet.

7. Verfahren gemäß einem der Ansprüche 1 bis 6, wobei in Schritt i) die Acetylierung mit Hilfe von Acetylchlorid in Anwesenheit einer Lewis-Säure und Dichlormethan erfolgt.

8. Verfahren gemäß einem der Ansprüche 1 bis 7, wobei in Schritt (i-a) die sauren Bedingungen mit Hilfe einer anorganischen Säure hergestellt werden.

9. Verbindung der Formel (1) erhältlich durch ein Verfahren gemäß einem der Ansprüche 1 bis 8 welche Kaempferol und/oder dessen Derivate zu weniger als 0,5 % aufweist.

10. Verbindung gemäß Anspruch 9, wobei R¹, R², R³, R⁴, R⁵ und R⁶ Wasserstoff sind.

## Claims

1. A method for producing a compound represented by the following formula (1), 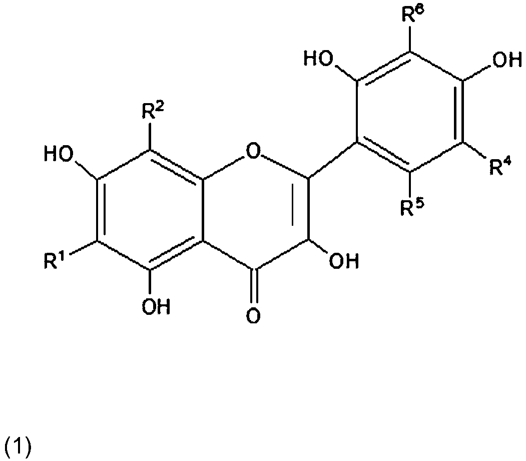 comprising the steps of:
i) acetylating a compound of the following formula (4) to form the 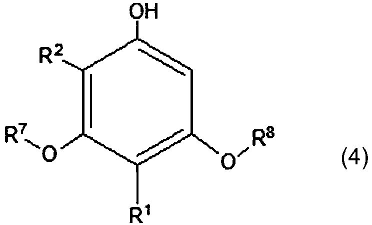 acetophenone of formula (6), 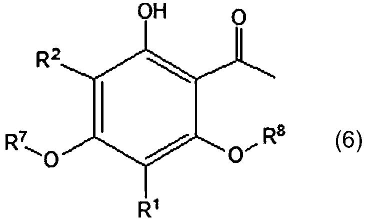
ii) reacting the acetophenone of formula (6) with a compound of the following formula (7) under basic conditions 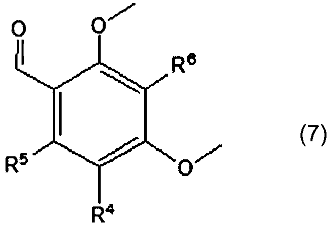 at room temperature to form the chalcone of the following formula (8), 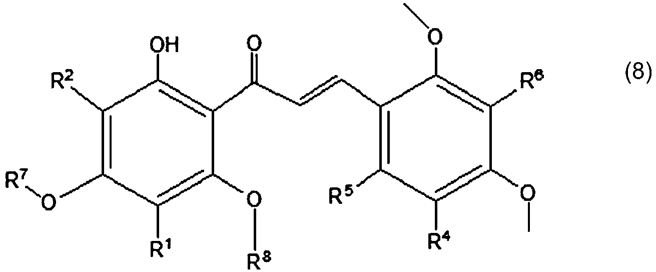
iii) Reacting the chalcone of formula (8) under oxidizing conditions in a basic medium to form a flavonol of the following formula (9); 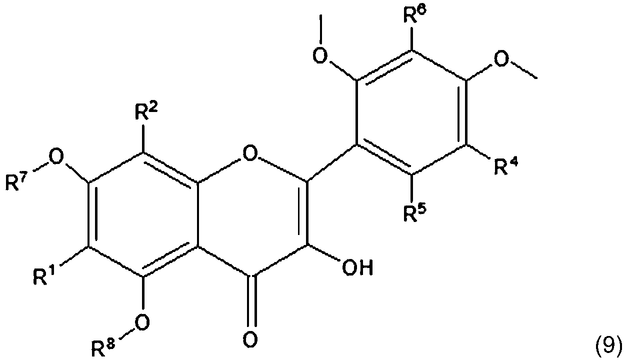
iv) and demethylating the flavonol with formula (9),
where
R¹, R², R³, R⁴, R⁵ and R⁶ are a branched or unbranched C₁-C₈₋alkyl, NO₂, SO₃H, NX₂ , wherein X is an ethyl or methyl radical or hydrogen, CF₃ or hydrogen, preferably C₁-C₅ alkyl or hydrogen, most preferably C₁-C₃ alkyl or hydrogen, and
R⁷ and R⁸ independently represent a methyl, ethyl, t-butyl, benzyl, methoxymethyl, p-methoxybenzyl, benzyloxymethyl, triphenylmethyl, tetrahydropyranyl, or allyl group.

2. The method according to claim 1, further comprising step (i-a):
(i-a) Methylation of a compound of the following formula (3) 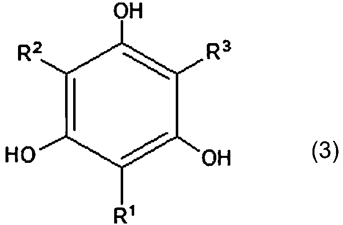
under acidic conditions to a compound of formula (4),
wherein R¹, R², R³, R⁴, R⁵ and R⁶ are as defined in claim 1.

3. Method according to any one of claims 1 to 2, wherein R³ is hydrogen.

4. Method according to any one of claims 1 to 3, wherein R¹ , R² , R³ , R⁴ , R⁶ and R⁶ are hydrogen.

5. Method according to any one of claims 1 to 4, wherein in step iii) the oxidizing conditions are established using tert-butyl hydroperoxide and the basic environment is established using an alkali hydroxide.

6. Method according to one of claims 1 to 5, wherein in step ii) the basic conditions are established with the aid of an alkali hydroxide and the reaction takes place at room temperature.

7. Method according to one of claims 1 to 6, wherein in step i) the acetylation is carried out using acetyl chloride in the presence of a Lewis acid and dichloromethane.

8. Methode according to one of claims 1 to 7, wherein in step (i-a) the acidic conditions are established with the aid of an inorganic acid.

9. Compound of formula (1) obtainable by a method according to one of claims 1 to 8, which has less than 0.5% kaempferol and/or its derivatives.

10. Compound according to claim 9, wherein R¹, R², R³, R⁴, R⁵ and R⁶ are hydrogen.

## Revendications

1. Procédé de préparation d'un composé représenté par la formule (1) suivante, 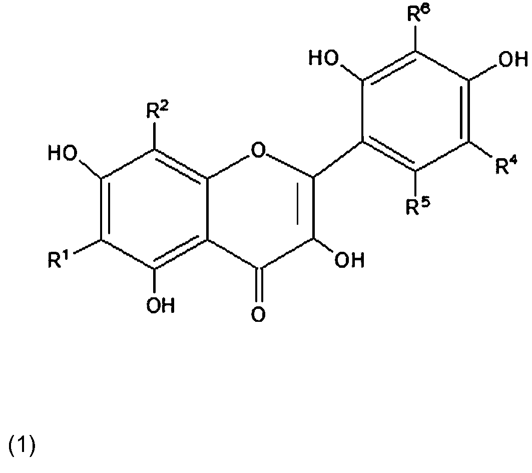 comprenant les étapes suivantes :
i) l'acétylation d'un composé représenté par la formule (4) suivante pour former le 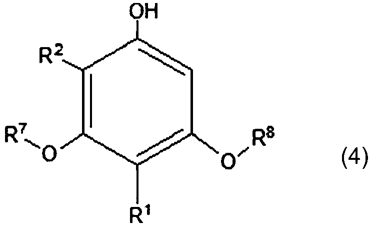 acétophénone de formule (6), 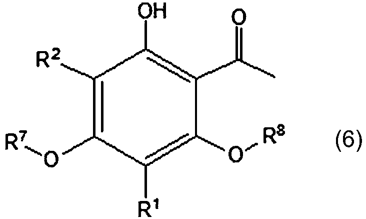
ii) Réaction de l'acétophénone de formule (6) avec un composé de formule (7) suivante dans des conditions basiques 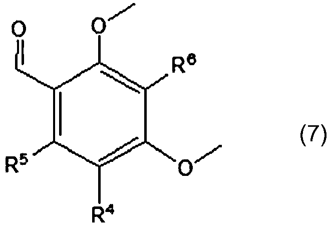 à température ambiante pour former la chalcone de formule (8) suivante, 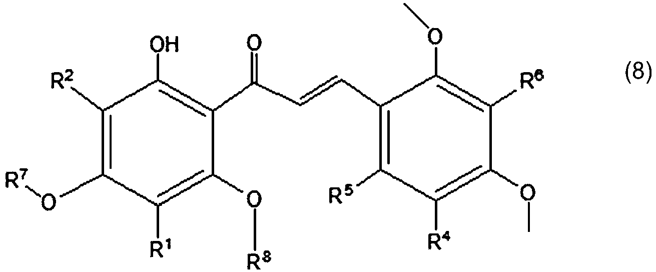
iii) Réaction de la chalcone de formule (8) dans des conditions oxydantes en milieu basique pour former un flavonol de formule suivante (9) ; 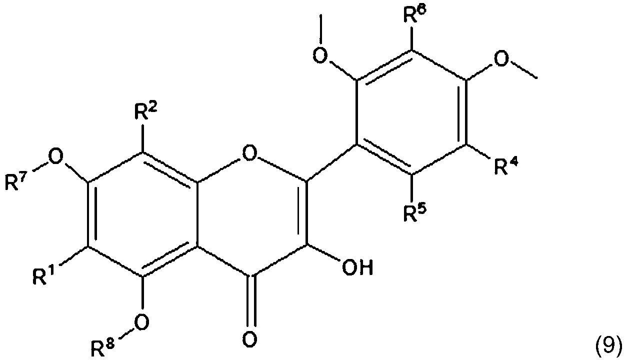
iv) et déméthylation du flavonol de formule (9),
où
R¹ , R² , R³ , R⁴ , R⁵ et R⁶ représentent un groupe alkyle en C₁-C₈ ramifié ou non ramifié, NO₂ , SO₃H , NX₂ , où X est un radical éthyle ou méthyle ou un atome d'hydrogène, CF₃ ou un atome d'hydrogène, de préférence un groupe alkyle en C₁ -C₅ ou un atome d'hydrogène, de préférence encore un groupe alkyle en C₁-C₃ ou un atome d'hydrogène, et
R⁷ et R⁸ sont indépendamment l'un de l'autre un groupe méthyle, éthyle, t-butyle, benzyle, méthoxyméthyle, p-méthoxybenzyle, benzyloxyméthyle, triphénylméthyle, tétrahydropyranyle ou allyle.

2. Procédé selon la revendication 1, comprenant en outre l'étape (i-a) :
(i-a) méthylation d'un composé de formule (3) suivante 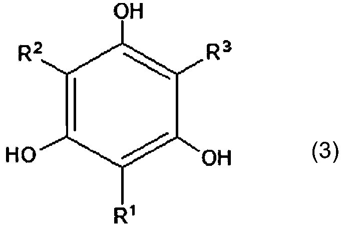
dans des conditions acides pour obtenir un composé de formule (4)
où R¹ , R² , R³ , R⁴ , R⁵ et R⁶ sont tels que définis dans la revendication 1.

3. Procédé selon l'une des revendications 1 à 2, dans lequel R³ est un atome d'hydrogène.

4. Procédé selon l'une des revendications 1 à 3, dans lequel R¹ , R² , R³ , R⁴ , R⁵ et R⁶ sont l'hydrogène.

5. Procédé selon l'une des revendications 1 à 4, dans lequel, à l'étape iii), les conditions oxydantes sont établies à l'aide d'hydroperoxyde de tert-butyle et le milieu basique est établi à l'aide d'un hydroxyde alcalin.

6. Procédé selon l'une des revendications 1 à 5, dans lequel, à l'étape ii), les conditions basiques sont établies à l'aide d'un hydroxyde alcalin et la réaction se déroule à température ambiante.

7. Procédé selon l'une des revendications 1 à 6, dans lequel, à l'étape i), l'acétylation est réalisée à l'aide de chlorure d'acétyle en présence d'un acide de Lewis et de dichlorométhane.

8. Procédé selon l'une des revendications 1 à 7, dans lequel, à l'étape (i-a), les conditions acides sont établies à l'aide d'un acide inorganique.

9. Composé de formule (1) obtenable par un procédé selon l'une des revendications 1 à 8, qui contient moins de 0,5 % de kaempférol et/ou de ses dérivés.

10. Composé selon la revendication 9, dans lequel R¹ , R² , R³ , R⁴ , R⁵ et R⁶ sont des atomes d'hydrogène.
